(19) Europäisches Patentamt
European Patent Office
Office européen des brevets

(11) EP 2 695 613 A1

(12) **EUROPEAN PATENT APPLICATION**
published in accordance with Art. 153(4) EPC

(43) Date of publication:
**12.02.2014 Bulletin 2014/07**

(21) Application number: **12742101.4**

(22) Date of filing: **30.01.2012**

(51) Int Cl.:
***A61K 31/201*** *(2006.01)* ***A61P 29/00*** *(2006.01)*

(86) International application number:
**PCT/JP2012/051939**

(87) International publication number:
**WO 2012/105477 (09.08.2012 Gazette 2012/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.01.2011 JP 2011018925**

(71) Applicants:
- **Nippon Zoki Pharmaceutical Co., Ltd.**
**Osaka-shi**
**Osaka 541-0046 (JP)**
- **Nagoya Industrial Science Research Institute**
**Nagoya-shi**
**Aichi 460-0008 (JP)**

(72) Inventors:
- **IINUMA, Munekazu**
**Gifu-shi**
**Gifu 501-1196 (JP)**
- **FURUKAWA, Shoei**
**Gifu-shi**
**Gifu 501-1196 (JP)**
- **NAIKI, Mitsuru**
**Kato-shi**
**Hyogo 673-1461 (JP)**
- **OKADA, Tomoyuki**
**Kato-shi**
**Hyogo 673-1461 (JP)**
- **MATSUMOTO, Tomonori**
**Kato-shi**
**Hyogo 673-1461 (JP)**
- **SAWADA, Kazuyoshi**
**Kato-shi**
**Hyogo 673-1461 (JP)**

(74) Representative: **HOFFMANN EITLE**
**Patent- und Rechtsanwälte**
**Arabellastrasse 4**
**81925 München (DE)**

(54) **ANALGESIC**

(57) An object of the present invention is to provide an analgesic effective in a prophylactic or a therapy for various pain diseases. The present invention is to provide an analgesic containing an ester of $C_{10}$ fatty acid as an active ingredient. The analgesic of the present invention containing the compound as an active ingredient is highly useful as a prophylactic or therapeutic agent for various pain diseases such as the pain caused by osteoarthritis (e.g., knee osteoarthritis and hip osteoarthritis) or by demyelinating diseases such as multiple sclerosis or Guillain-Bareé syndrome.

[Fig. 1]

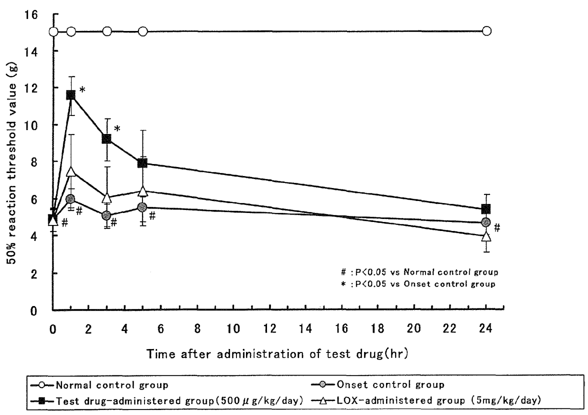


EP 2 695 613 A1

## Description

### [Technical Field]

The present invention relates to a novel pharmaceutical use of an ester of $C_{10}$ fatty acid and, more particularly, it relates to an analgesic containing an ester of $C_{10}$ fatty acid as an active ingredient.

### [Background Art]

The present invention relates to an analgesic containing an ester of $C_{10}$ fatty acid as an active ingredient. As to the ester of $C_{10}$ fatty acid, 10-hydroxyl-2-decenoic acid has been widely known as a specific component of royal jelly and is a substance having an insulin-like action and a normalizing action for sugar metabolism in the body whereby it is expected for its improvement in a lifestyle-related disease and for its beauty effect. However, 10-hydroxyl-2-decenoic acid is a compound having a different structure from the ester of $C_{10}$ fatty acid which is an active ingredient of the drug of the present invention in such a respect that its position 10 is substituted with hydroxyl group and that it is not an ester. Meanwhile, Patent Document 1 discloses that a $C_8$ or $C_{10\sim12}$ fatty acids as well as ester thereof has/have a neurotrophic factor-like activity. However, with regard to the agent having a neurotrophic factor-like activity in the Patent Document 1, there is a mere description that it is useful as a prophylactic/improving agent for neurodegenerative disease such as Alzheimer disease or Parkinson disease and for mental disease such as depression or anxiety disorder (neurosis) and there is no description therein at all that an analgesic action as in the present invention is available. As mentioned above, it is the finding being unknown up to now that an ester of $C_{10}$ fatty acid is effective as an analgesic for pain diseases.

### [Prior Art Documents]

### [Patent Document]

Patent Document 1: International Publication No. WO 2009/038110

### [Summary of the Invention]

### [Problems to be solved by the Invention]

An object of the present invention is to provide an analgesic having an excellent effect.

### [Means for Solving the Problems]

As a result of intensive studies, the present inventors have found that an ester of $C_{10}$ fatty acid has an excellent analgesic effect to a pain diseases and accomplished the present invention. Thus the present invention is as follows.

(1) An analgesic containing an ester of $C_{10}$ fatty acid as an active ingredient.
(2) The analgesic according to (1), wherein the fatty acid in the ester of fatty acid is decenoic acid.
(3) The analgesic according to (2), wherein the decenoic acid is 2-decenoic acid.
(4) The analgesic according to (3), wherein the 2-decenoic acid is trans-2-decenoic acid.
(5) The analgesic according to any of (1) to (4), wherein the ester in the ester of fatty acid is an alkyl ester.
(6) The analgesic according to any of (1) to (4), wherein the ester in the ester of fatty acid is an alkenyl ester.
(7) The analgesic according to any of (1) to (4), wherein the ester in the ester of fatty acid is a cycloalkyl ester.
(8) The analgesic according to any of (1) to (7), wherein the analgesic is a therapeutic agent for arthralgia.
(9) The analgesic according to (8), wherein the arthralgia is the pain caused by osteoarthritis.
(10) The analgesic according to (9), wherein the osteoarthritis is knee osteoarthritis or hip osteoarthritis.
(11) The analgesic according to any of (1) to (7), wherein the analgesic is a therapeutic agent for pain accompanied by demyelinating disease.
(12) The analgesic according to (11), wherein the demyelinating disease is multiple sclerosis or Guillain-Bareé syndrome.
(13) The analgesic according to any of (1) to (12), wherein the analgesic is an injectable preparation.
(14) The analgesic according to any of (1) to (12), wherein the analgesic is an oral preparation.
(15) The analgesic according to any of (1) to (12), wherein the analgesic is a cyclodextrin inclusion complex.
(16) The analgesic according to any of (1) to (12), wherein the analgesic is an external preparation.
(17) The analgesic according to (16), wherein the external preparation is a patch preparation.

(18) The ester of $C_{10}$ fatty acid according to any of (1) to (7) for use in the therapy of pain diseases.
(19) A method for treating pain diseases, comprising administering the ester of $C_{10}$ fatty acid ester according to any of (1) to (7) in effective dose to a patient suffering from pain diseases.
(20) Use of the ester of $C_{10}$ fatty acid according to any of (1) to (7) in the manufacture of a drug for the therapy of pain diseases.

**[Advantages of the Invention]**

The ester of $C_{10}$ fatty acid in accordance with the analgesic of the present invention is a compound having an excellent analgesic action and is very useful as a drug for the therapy of various pain diseases including the pain by arthralgia such as osteoarthritis and the pain accompanied by demyelinating disease such as multiple sclerosis or Guillain-Bareé syndrome.

**[Brief Description of Drawings]**

[Fig. 1] Fig. 1 is the result testing the effect of a single administration of the analgesic of the present invention to hyperalgesia of osteoarthritis model rats.
[Fig. 2] Fig. 2 is the result testing the effect of repeated administrations of the analgesic of the present invention to hyperalgesia of osteoarthritis model rats.
[Fig. 3] Fig. 3 is the result testing the effect of repeated administrations of the analgesic of the present invention to arthralgia of osteoarthritis model rats.
[Fig. 4] Fig. 4 is the result testing the effect of repeated administrations of the analgesic of the present invention to hyperalgesia of osteoarthritis model rats in the same manner as in Fig. 3.

**[Mode for Carrying Out the Invention]**

The present invention relates to an analgesic containing an ester of $C_{10}$ fatty acid as an active ingredient.
The ester of fatty acid which is able to be utilized as an active ingredient of the analgesic of the present invention is an ester of fatty acid comprising a $C_{10}$ fatty acid and an alcohol, and the ester of fatty acid as such may be used solely or two or more thereof may be used in combination. Although the $C_{10}$ fatty acid may be any of decanoic acid (caprylic acid) which is a linear saturated fatty acid, decenoic acid which is a linear unsaturated fatty acid and geranic acid which is a branched unsaturated fatty acid, a preferred one is an unsaturated fatty acid having one double bond of carbons such as 2-decenoic acid, 3-decenoic acid or 9-decenoic acid, more preferred one is a trans compound thereof and the particularly preferred one is trans-2-decenoic acid.
On the other hand, with regard to the alcohol forming an ester moiety of the fatty acid ester in the analgesic of the present invention, there may be exemplified an alkyl alcohol, an alkenyl alcohol and a cycloalkyl alcohol.
Although there is no particular limitation for the alkyl alcohol, a preferred one is an alcohol having a linear or branched alkyl having 1 to 12 carbon(s) such as methyl, ethyl, propyl, isopropyl, butyl, isobutyl, sec-butyl, tert-butyl, pentyl, isopentyl, neopentyl, tert-pentyl, hexyl, isohexyl, heptyl, isoheptyl, octyl, isooctyl, nonyl, isononyl, decyl, isodecyl, undecyl, isoundecyl, dodecyl or isododecyl.
Although there is no particular limitation for the alkenyl alcohol, a preferred one is an alcohol having a linear or branched alkenyl having 2 to 12 carbons such as ethenyl, propenyl, isopropenyl, butenyl, isobutenyl, sec-butenyl, tert-butenyl, pentenyl, isopentenyl, neopentenyl, tert-pentenyl, hexenyl, isohexenyl, heptenyl, isoheptenyl, octenyl, isooctenyl, nonenyl, isononenyl, decenyl, isodecenyl, undecenyl, isoundecenyl, dodecenyl or isododecenyl and a more preferred one is an alcohol having a linear or branched alkenyl having 9 to 11 carbons such as nonenyl, isononenyl, decenyl, isodecenyl, undecenyl or isoundecenyl.
Although there is no particular limitation for the cycloalkyl alcohol, a preferred one is an alcohol having a cycloalkyl having 3 to 8 carbons such as cyclopropyl, cyclobutyl, cyclopentyl, cyclohexyl, cycloheptyl or cyclooctyl and a more preferred one is an alcohol having a cycloalkyl having 5 or 6 carbons such as cyclopentyl or cyclohexyl.
The known compounds mentioned in Patent Document 1 or commercially available compounds can be used as the ester of $C_{10}$ fatty acid which is an active ingredient of the analgesic in the present invention. The ester of $C_{10}$ fatty acid can also be produced by known methods, for example, by subjecting a $C_{10}$ fatty acid and an alcohol to a dehydration-condensation. The dehydration-condensation reaction may adopt the conventionally known methods.
As a known condensation method, for example, a $C_{10}$ fatty acid may be made to react with an alcohol in the presence of an appropriate condensing agent (such as dicyclohexylcarbodiimide (DCC) or N-(3-dimethylaminopropyl)-N'-ethylcarbodiimide·HCl). The reaction may be usually carried out in a common solvent (such as dichloromethane).

Usually, the using amount of the alcohol is 0.5 to 2 mol (preferably, 1 to 1.5 mol) to 1 mol of the $C_{10}$ fatty acid.

Alternatively, a $C_{10}$ fatty acid may be, for example, once converted to a carboxylic halide and then made to react with an alcohol in the presence or absence of a base. Conversion to the carboxylic halide may be carried out, for example, using a halogenating agent such as thionyl chloride, sulfyryl chloride, phosphorus trichloride, phosphorus pentachloride, oxalyl chloride or phosphoric acid trichloride. Examples of the base include triethylamine and pyridine. Usually, the using amount of the alcohol is 0.5 to 2 mol (preferably, 1 to 1.5 mol) to 1 mol of the $C_{10}$ fatty acid. When a base is used, the using amount of the base is usually about 1 to 5 mol to 1 mol of the $C_{10}$ fatty acid.

Examples of the compound produced as such are shown in Tables 1 and 2. When each compound is referred to hereinafter, the compound number mentioned in the tables is used.

[Table 1]

| Compound No. | Compound Name | Structural Formula |
|---|---|---|
| 1 | Decanoic acid methyl ester | 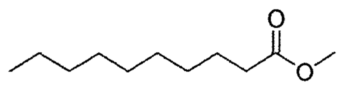 |
| 2 | Decanoic acid ethyl ester | 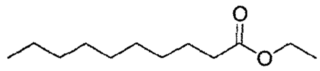 |
| 3 | Trans-2-decenoic acid methyl ester | 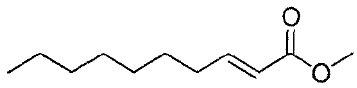 |
| 4 | Trans-2-decenoic acid ethyl ester | 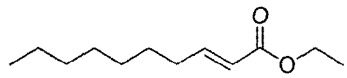 |
| 5 | Trans-2-decenoic acid propyl ester | 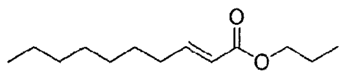 |
| 6 | Trans-2-decenoic acid isopropyl ester | 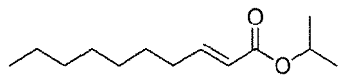 |
| 7 | Trans-2-decenoic acid butyl ester | 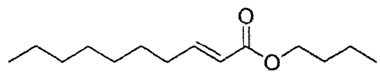 |
| 8 | Trans-2-decenoic acid *sec*-butyl ester | 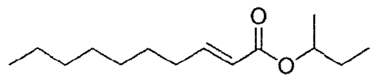 |
| 9 | Trans-2-decenoic acid isobutyl ester | 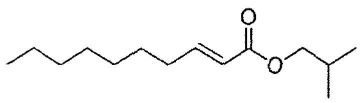 |
| 10 | Trans-2-decenoic acid *tert*-butyl ester | 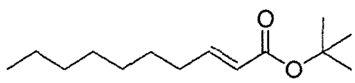 |
| 11 | Trans-2-decenoic acid hexyl ester | 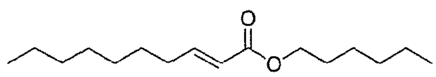 |
| 12 | Trans-2-decenoic acid heptyl ester | 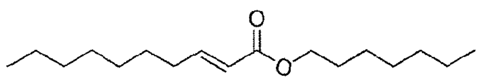 |
| 13 | Trans-2-decenoic acid octyl ester | 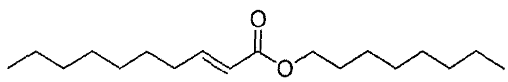 |
| 14 | Trans-2-decenoic acid decyl ester | 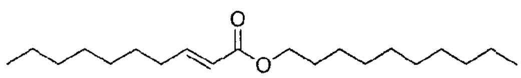 |
| 15 | Trans-2-decenoic acid undecyl ester | 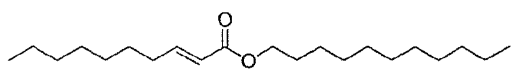 |

[Table 2]

| Compound No. | Compound Name | Structural Formula |
|---|---|---|
| **16** | Trans-2-decenoic acid trans-2-decenyl ester | 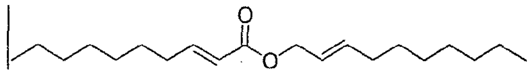 |
| **17** | Trans-2-decenoic acid trans-9-decenyl ester | 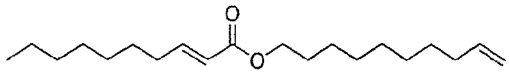 |
| **18** | Trans-2-decenoic acid cyclohexyl ester | 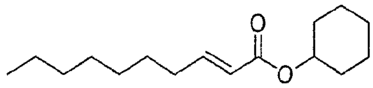 |
| **19** | Trans-3-decenoic acid methyl ester | 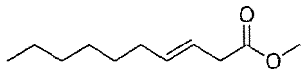 |
| **20** | Trans-3-decenoic acid ethyl ester | 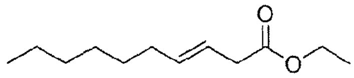 |
| **21** | Trans-9-decenoic acid methyl ester | 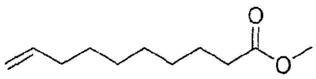 |
| **22** | Trans-9-decenoic acid ethyl ester | 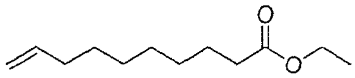 |
| **23** | Geranic acid ethyl ester | 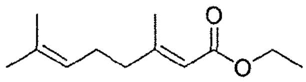 |

With regard to the compounds 8, 9, 12, 14 15 an 17 which have not been mentioned in the documents yet among the compounds mentioned in Tables 1 and 2, they were synthesized by means of the above-mentioned reaction using trans-2-decenoic acid and an appropriate alcohol corresponding to each ester moiety and were purified using silica gel chromatography or the like whereupon each of the compounds was produced as an oily substance.

The analgesic of the present invention contains an ester of $C_{10}$ fatty acid as an active ingredient and is useful as a prophylactic or therapeutic agent for various pain diseases. Examples of the pain diseases include arthralgia such as the pain by osteoarthritis (e.g., knee osteoarthritis and hip osteoarthritis) or by rheumatoid arthritis and the pain accompanied by demyelinating diseases such as multiple sclerosis or Guillain-Bareé syndrome.

The ester of $C_{10}$ fatty acid of the present invention can be made into a pharmaceutical preparation in various dosage forms (such as oral, injectable and external preparations) by appropriately combining with an appropriate pharmaceutical carrier or diluent. The drug of the present invention may be also a combination drug in which the ester of $C_{10}$ fatty acid is combined with other pharmaceutically active ingredient(s). Further, the analgesic of the present invention may be made into a preparation as a cyclodextrin inclusion complex or the like. As a result, there may be the cases where enhancement of pharmacological activity, improvement in stability, prolonged action, easy handling, etc. can be achieved. An inclusion complex can be formed by, for example, mixing an ester of $C_{10}$ fatty acid with α-, β- or γ-cyclodextrin whereby enhancement of pharmacological activity upon, for example, oral administration is noted.

When the analgesic of the present invention is made into an oral preparation, it is possible to make into tablet, powder, granule or capsule preparation by means of such a formulation where the ester of $C_{10}$ fatty acid is appropriately combined with an appropriate additive such as excipient, binder, disintegrator, lubricant, extender, wetting agent, buffer, preservative or flavoring. In making into an injectable preparation, it is possible to make into an injectable preparation by addition of stabilizer, preservative, isotonic agent or the like to a solution or suspension containing the ester of $C_{10}$ fatty acid. In making into an external preparation, it is possible to make into an external preparation such as patch preparation, gel preparation, ointment, cream preparation or the like. Thus, the ester of $C_{10}$ fatty acid is, for example, mixed with, melted in or emulsified in an appropriate base and, in the case of a patch preparation, the above is spread and applied onto a support. In the case of a patch preparation, a gel preparation or the like, it can be made, for example, into a composition using an organogelling agent. Incidentally, depending upon the dosage form of each external preparation, a commonly used preservative, antioxidant, flavoring agent, adhesive or the like may be appropriately selected and added to a formulation.

Adequate dose of the compound of the present invention may be appropriately increased or decreased by

taking dose regimen, age, sex, symptom in a patient, etc. into consideration and, may be generally administered in an amount of from 1 to 1,000 mg or, preferably, 5 to 300 mg, for adult, at ounce or in several divided administrations per day.

**[Examples]**

Hereinafter, an example of results of a pharmacological test concerning novel pharmacological activity of the ester of $C_{10}$ fatty acid of the present invention, that is, an analgesic effect, is described. The present invention is not intended to be limited to the descriptions in Examples.

Pharmacological Test I: Analgesic action to osteoarthritis model rats

There were conducted the following experiments for testing the analgesic action of the analgesic of the present invention using the osteoarthritis (OA) rats induced by sodium monoiodoacetate (MIA) which were model animals for OA.

1. Single administration studies of the analgesic of the present invention to MIA-induced OA rats

(1) Preparation of MIA-induced OA rats

A 50% reaction threshold value to the mechanical stimulus of male Wistar rats of six weeks age was measured (the measuring method will be mentioned later) and a normal control group was selected. MIA prepared by saline was administered in a single dose of 0.3 mg/50 μL into right knee joint of the rats except the normal control group while 50 μL of saline was administered into left knee joint whereupon the MIA-induced OA rats were prepared. To the normal control group, 50 μL of saline was administered into the joints of both knees.

(2) Grouping

With regard to the male Wistar rats of six weeks age used as experimental animals except the normal control group, their 50% reaction threshold values to the mechanical stimulus (the measuring method will be mentioned later) and body weights were measured after 24 days from the administration of MIA mentioned in (1) and then 4 groups each comprising six rats [a normal control group, an onset control group, a test drug-administered group and a loxoprofen sodium hydrate (LOX)-administered group (a positive control)] were organized.

(3) Administration of test drug

A test drug solution (0.1 mg/mL) using the compound 4 as a test drug and a LOX suspension (1 mg/mL) were prepared using a phosphate buffered saline (PBS) containing 0.1 vol% of dimethyl sulfoxide (DMSO) and a 0.5 w/v% aqueous solution of carboxymethyl cellulose, respectively.

Immediately after the grouping (after 24 days from the administration of MIA), a test drug solution was intraperitoneally administered in a single dose of 0.5 mg/kg to a test drug-administered group while, a LOX suspension was orally administered in a single dose of 5 mg/kg to a LOX-administered group. Meanwhile, to the normal control group and the onset control group, PBS containing 0.1 vol% of DMSO was intraperitoneally administered in a single dose.

(4) Result of measurement of the 50% reaction threshold values to the mechanical stimulus (von Frey test)

The four groups of rats of the above (2) were placed in a transparent acrylic cage with a wire-meshed floor and habituated for about three minutes and the 50% reaction threshold values to the mechanical stimulus were measured after 1, 3, 5 and 24 hours from the administration of a test drug.

The measurement was conducted using von Frey filaments (manufactured by North Coast Medical Inc.) in accordance with the methods of Chaplan, et al. (Journal of Neuroscience Methods, vol. 53, no. 1, pages 55 to 63, 1994) and Dixon, et al. (Annual Review of Pharmacology and Toxicology, vol. 20, pages 441 to 462, 1980). In eight filaments [stimulus loads (g): 0.4, 0.6, 1.0, 2.0, 4.0, 6.0, 8.0 and 15.0], the test was started as from the filament of 2.0 g, the filament was vertically attached to the sole for 2 to 3 seconds with such a force that the filament was lightly bent and the case where the hind limb showed an escape reaction was called a positive reaction. The case where the rat escaped at the instance of removing the filament was also called positive. When the positive reaction was noted, stimulus was conducted similarly using a filament of one rank weaker while, when no reaction was noted, stimulus was conducted similarly using a filament of one rank stronger and the point when the reaction changed from negative to positive or from positive to negative was called the first two reactions. After that, stimulus was conducted for continuous four times by the same up-down method. A 50% reaction threshold value to the mechanical stimulus was measured using the reaction to the six stimuli in total and then (mean value) ± (standard error) for each group was calculated. Incidentally, when stimulus reached by that of 15.0 g without positive reaction or, when positive reaction continued to 0.4 g, then 15.0 g or 0.25 g

was adopted as each threshold value, respectively.

The test for significant difference was performed using the test in two groups (Student's t-test or Welch test) or Wilcoxon test between the normal control group and the onset control group, between the onset control group and the test drug-administered group, between the onset control group and the LOX-administered group and between the test drug-administered group and the LOX-administered group. Analysis was conducted using SAS System Version 8.2 (SAS preclinical package Ver. 5.0, SAS Institute Japan) and, it was judged that $p<0.05$ is significantly different.

An example of the results of the above test is shown in Fig. 1. The 50% reaction threshold value to the mechanical stimulus in the onset control group where OA was induced by administration of MIA significantly lowered as compared with the normal control group. On the contrary, in the test drug-administered group where the test drug (the compound 4) was intraperitoneally administered in a single dose after administration of MIA, significantly high 50% reaction threshold values as compared with the onset comparative group were noted as shown in Fig. 1. Although no significant suppressive action to hyperalgesia was noted in the 5 mg/kg of LOX-administered group, a dose of as low as 0.5 mg/kg showed a suppressive effect for hyperalgesia in the test drug-administered group.

An example of the results of the test that the analgesic of the present invention was administered in a single dose in the same manner as described above is shown in Fig. 3. A von Frey test was conducted in the same manner as described above and each 50% reaction threshold value was measured. With regard to the 50% reaction threshold value after 1 hour from administration of a test drug, a recovery rate (%) of the 50% reaction threshold value was calculated.

Recovery rate (%) of 50% reaction threshold value = {[(50% reaction threshold value after 1 hour from administration of test drug) - (50% reaction threshold value before administration of test drug)] ÷ [(Normal threshold value) - (50% reaction threshold value before administration of test drug)]} × 100

[Table 3]

| Test drug | Recovery rate of 50% reaction threshold value (%) |
|---|---|
| Compound 3 | 32.4 |
| Compound 4 | 61.6 |
| Compound 5 | 8.1 |
| Compound 16 | 39.6 |
| Compound 17 | 19.5 |
| Compound 18 | 20.8 |
| Compound 20 | 17.7 |

As will be apparent from Table 3, the analgesic of the present invention showed an excellent suppressive effect to hyperalgesia of OA induced by the administration of MIA. Incidentally, in the case of trans-2-decenoic acid which is not an ester, there was no difference in the 50% reaction threshold values between the stages before the administration of the test drug and after 1 hour from the administration of the test drug whereby no suppressive effect for hyperalgesia was noted.

2. Repeated administration studies of the analgesic of the present invention to MIA-induced OA rats [1]

(1) Preparation of MIA-induced OA rats

Selection of the normal control group and preparation of MIA-induced OA rats were conducted in the same manner as in the above 1.(1).

(2) Grouping

With regard to the male Wistar rats of six weeks age used as experimental animals except the normal control group, their 50% reaction threshold values to the mechanical stimulus, weight bearing rate to a MIA-non-administered hind paw (the measuring method will be mentioned later) and body weights were measured after 27 days from the administration of MIA mentioned in (1) and then four groups each comprising six rats [a normal control group, an onset control group, a test drug-administered group and a LOX-administered group (a positive control)] were organized.

(3) Administration of test drug

A test drug solution and an LOX suspension were prepared in the same manner as in the above 1.(2).

During eight days from immediately after the grouping (after 27 to 34 days from the administration of MIA), a test drug solution was intraperitoneally administered in repeated dose of 0.5 mg/kg to a test drug-administered group while, a LOX suspension was orally administered in repeated dose of 5 mg/kg to a LOX-administered group. Meanwhile, to the normal control group and the onset control group, PBS containing 0.1 vol% of DMSO was intraperitoneally administered repeatedly.

(4) Result of measurement of the 50% reaction threshold values to the mechanical stimulus (von Frey test)

In the same manner as in the above 1.(4), the 50% reaction threshold values to the mechanical stimulus were measured after 1, 3, 5 and 24 hour(s) from the final administration of a test drug. Mean value ± standard error for each group was calculated and the test for significant difference was done in the same manner as in the above 1.(4).

An example of the results of the above test is shown in Fig. 2. The 50% reaction threshold value to the mechanical stimulus in the onset control group where OA was induced by administration of MIA significantly lowered as compared with the normal control group. On the contrary, as shown in Fig. 2, significantly high 50% reaction threshold values were noted after 1 and 3 hour(s) from the administration as compared with the onset control group in the test drug-administered group where the test drug (the compound 4) was intraperitoneally administered repeatedly after administration of MIA. As such it has been confirmed that an excellent suppressive effect for hyperalgesia is also available when the analgesic of the present invention is repeatedly administered.

(5) Measurement of weight bearing rate to a MIA-non-administered hind paw in a method using Dual channel weight averager

One hind paw of a rat where OA was induced by administration of MIA develops a pain symptom. Therefore, a body weight bearing to a painless hind paw to which no MIA was administered increases in a rat for avoiding the application of body weight to the hind paw after administration of MIA. However, when the pain symptom is improved by administration of a test substance, application of weight to the hind paw after administration of MIA becomes easier for a rat and a weight bearing rate to the MIA-non-administered hind paw decreases to such an extent. Using this weight bearing rate to the MIA-non-administered hind paw as an index, the analgesic action of the analgesic of the present invention was measured.

The weight bearing rate to the MIA-non-administered hind paw was determined by the following formula after measuring the weight distribution to right and left hind paws of a rat using a device for evaluating the analgesic effect for small animals (Incapacitance Tester manufactured by Linton Instrumentation). Mean value ± standard error for each group was also calculated and the test for significant difference was carried out in the same manner as in the above 1.(4).

Weight bearing rate (%) to MIA-non-administered hind paw = {[(Body weight bearing to MIA-non-administered hind paw) - (Body weight bearing to MIA-administered hind paw)] ÷ [(Body weight bearing to MIA-non-administered hind paw) + (Body weight bearing to MIA-administered hind paw)]} × 100

An example of the above test results is shown in Fig. 3. The weight bearing rate to MIA-non-administered hind paw of the onset control group where OA was induced by administration of MIA significantly increased as compared with the normal control group. On the contrary, in a test drug-administered group where the test drug (the compound 4) was intraperitoneally administered repeatedly after administration of MIA, the weight bearing rate to the MIA-non-administered hind paw significantly decreased as compared with the onset control group as shown in Fig. 3. As such, it has been confirmed that the analgesic of the present invention has an excellent analgesic effect to the pain by OA induced by administration of MIA.

3. Repeated administration studies of the analgesic of the present invention to MIA-induced OA rats [2]

(1) Measurement of the 50% reaction threshold values to the mechanical stimulus

The analgesic of the present invention was repeatedly administered to the MIA-induced OA rat and a von Frey test was conducted in the same manner as in the above test 1.(4) whereupon each 50% reaction threshold value was measured. With regard to the 50% reaction threshold value after 1 hour from the administration of a test drug, a recovery rate (%) of the 50% reaction threshold value was calculated in the same manner as in the above test 1.(4). An example of this test results is shown in Table 4.

[Table 4]

| Test drug | Recovery rate of 50% reaction threshold value (%) |
|---|---|
| Compound 3 | 23.3 |
| Compound 16 | 61.4 |
| Compound 18 | 32.6 |
| Compound 20 | 66.3 |

As will be apparent from Table 4, the analgesic of the present invention showed an excellent suppressive effect to hyperalgesia of OA induced by administration of MIA.

(2) Measurement of weight bearing rate to a MIA-non-administered hind paw in a method using Dual channel weight averager

The analgesic of the present invention was intraperitoneally administered in repeated dose of 0.5 mg/kg/day to the MIA-induced OA rat and the weight bearing rate to the MIA-non-administered hind paw was measured in the same manner as in the above test 2.(5). An example of the test results thereof is shown in Fig. 4.

As will be apparent from Fig. 4, the analgesic of the present invention (the compound 16) significantly lowered the weight bearing rate and, in other compounds, the weight bearing rate was also decreased. Therefore, it has been confirmed that the analgesic of the present invention has an analgesic effect to the pain of OA induced by administration of MIA.

Pharmacological Test II: Analgesic action to multiple sclerosis model rats

The following experiments were conducted for checking the analgesic action of the analgesic of the present invention using experimental autoallergic encephalomyelitis (EAE) rats which were the model animals for multiple sclerosis (MS) having an onset of chronic pain.

(1) Preparation of EAE rats

The 50% reaction threshold values to the mechanical stimulus of female Lewis rats of seven weeks age were measured whereupon a normal control group was selected. Left hind paw of each of the anesthetized rats except the normal control group was immunized by subcutaneous administration of 0.1 mL of an emulsion prepared by equivalent mixing of 0.4 mg/mL solution of a peptide derived from myelin basic protein (MB 68-84) of guinea pig with an adjuvant complete Freund H37Ra whereupon EAE rats were prepared.

(2) Grouping

Female Lewis rats of seven weeks age which were the experimental animals were divided into four groups each comprising eight rats [a normal control group, an onset control group, a 0.25 mg/kg/day of test drug-administered group and a 0.50 mg/kg/day of test drug-administered group].

(3) Administration of test drug

During the period of from the immunized day until 28 days thereafter, the compound 4 was intraperitoneally administered once daily in repeated dose of 0.25 mg/kg/day or 0.50 mg/kg/day to the test drug-administered group.

(4) Result of measurement of the 50% reaction threshold values to the mechanical stimulus (von Frey test)

A von Frey test was carried out in the same manner as in 1.(4) of the above Pharmacological Test I to measure the 50% reaction threshold value to the mechanical stimulus to each group over time and the recovery rate (%) of the 50% reaction threshold value was calculated according to the following formula. The test for significant difference was conducted using the test in two groups (t-test or Welch test) between the normal control group and the onset control group. Between the onset control group and the test drug-administered group, it was conducted using the nonparametric or parametric Dunnett multiple comparison test. Analysis was conducted using SAS System Version 8.2 (SAS preclinical package Ver. 5.0, SAS Institute Japan) and, it was judged that $p<0.05$ is significantly different.

Incidentally, since the present EAE rats showed a clinical symptom (paralysis) due to demyelinating degeneration during the period of from 11 days to 19 days after the immunized day, no evaluation of the analgesic action by the 50% reaction threshold value to the mechanical stimulatus was conducted during that period.

Recovery rate (%) of 50% reaction threshold value = {[(50% reaction threshold value of test drug-administered group)·(50% reaction threshold value of onset control group)] ÷ [(50% reaction threshold value of normal control group) - (50% reaction threshold value of onset control group)]} × 100

An example of the results of this test is shown in Table 5.

[Table 5]

| Test group | Recovery rate of 50% reaction threshold value (%) | | | |
|---|---|---|---|---|
| | After 7 days | After 22 days | After 26 days | After 28 days |
| 0.25 mg/kg/day of test drug-administered group | 26.3 | 37.3※ | 52.3※ | 17.4 |
| 0.50 mg/kg/day of test drug-administered group | 52.0 | 51.7※ | 50.2※ | 70.2※ |
| ※ : P<0.05 | | | | |

In the above test, the onset control group which was immunized by administration of a peptide derived from myelin basic protein of guinea pig and an adjuvant complete Freund showed a significant decrease in the 50% reaction threshold value to the mechanical stimulus before and after the onset of the transient paralysis as compared with the normal control group. On the contrary, it will be apparent from Table 5 that, in a test drug-administered group where a test drug (the compound 4) was intraperitoneally administered after immunization repeatedly, an excellent recovery rate of the 50% reaction threshold value was noted. As such, it has been confirmed that the analgesic of the present invention has an excellent analgesic effect to the pain accompanied by EAE.

Pharmacological test III: Analgesic action to Guillain-Bareé syndrome model rats

The following experiment for checking the analgesic action of the analgesic of the present invention was conducted using the experimental autoimmune neuritis (EAN) rats which were the model animals for Guillain-Bareé syndrome having an on set of chronic pain.

(1) Preparation of EAN rats

The 50% reaction threshold value to mechanical stimulus of female Lewis rats of seven weeks age were measured to select a normal control group. Left hind paw of each of the anesthetized rats except the normal control group was immunized by subcutaneous administration of 0.1 mL of an emulsion prepared by equivalent mixing of a 2 mg/mL solution of a peptide derived from bovine P2 protein (SP-26) with an adjuvant complete Freund H37Ra whereupon EAN rats were prepared.

(2) Grouping and administration of test drug

The grouping was conducted and a test drug was administered in the same manner as in (2) and (3) of the Pharmacological Test II.

(3) Result of measurement of the 50% reaction threshold values to the mechanical stimulus (von Frey test)

A von Frey test was carried out in the same manner as in 1.(4) of the above Pharmacological Test I to measure the 50% reaction threshold value to the mechanical stimulus to each group over time. The test for significant difference was conducted and recovery rate (%) of the 50% reaction threshold value was calculated in the same manner as in (4) of the above Pharmacological Test II. Incidentally, since the present EAN rats showed a clinical symptom (paralysis) due to demyelinating degeneration during the period of from 12 days to 21 days after the immunized day, no evaluation of the analgesic by the 50% reaction threshold value to the mechanical stimulus was conducted during that period.

An example of the results of this test is shown in Table 6.

[Table 6]

| Test group | Recovery rate of 50% reaction threshold value (%) | | | |
|---|---|---|---|---|
| | After 8 days | After 11 days | After 25 days | After 28 days |
| 0.25 mg/kg/day of test drug-administered group | 100.0※ | 40.1※ | 21.7※ | 24.2※ |
| 0.50 mg/kg/day of test drug-administered group | 98.5※ | 35.5 | 38.3※ | 38.3※ |
| | | | | ※ : P<0.05 |

In the above test, the onset control group which was immunized by administration of a peptide derived from bovine P2 protein and an adjuvant complete Freund showed a significant decrease in the 50% reaction threshold value to the mechanical stimulation before and after the onset of the transient paralysis as compared with the normal control group. On the contrary, it will be apparent from Table 6 that, in a test drug-administered group where a test drug (the compound 4) was intraperitoneally administered repeatedly after immunization, an excellent recovery rate in the 50% reaction threshold value was noted. As such, it has been confirmed that the analgesic of the present invention has an excellent analgesic effect to the pain accompanied by EAN.

**[Industrial Applicability]**

As shown in the results of the above pharmacological tests, the analgesic of the present invention shows excellent analgesic effect and suppressive effect for hyperalgesia in animal experiments using MIA-induced OA rats which are an OA model and also in animal experiments using model rats for demyelinating diseases such as multiple sclerosis and Guillain-Bareé syndrome. Accordingly, the analgesic of the present invention is highly useful as a prophylactic or therapeutic agent for various pain diseases such as the pain caused by OA or demyelinating diseases.

## Claims

1. An analgesic containing an ester of $C_{10}$ fatty acid as an active ingredient.

2. The analgesic according to Claim 1, wherein a fatty acid in the ester of fatty acid is decenoic acid.

3. The analgesic according to Claim 2, wherein the decenoic acid is 2-decenoic acid.

4. The analgesic according to Claim 3, wherein the 2-decenoic acid is trans-2-decenoic acid.

5. The analgesic according to any of Claims 1 to 4, wherein an ester in the ester of fatty acid is an alkyl ester.

6. The analgesic according to any of Claims 1 to 4, wherein an ester in the ester of fatty acid is an alkenyl ester.

7. The analgesic according to any of Claims 1 to 4, wherein an ester in the ester of fatty acid is a cycloalkyl ester.

8. The analgesic according to any of Claims 1 to 7, wherein the analgesic is a therapeutic agent for arthralgia.

9. The analgesic according to Claim 8, wherein the arthralgia is the pain caused by osteoarthritis.

10. The analgesic according to Claim 9, wherein the osteoarthritis is knee osteoarthritis or hip osteoarthritis.

11. The analgesic according to any of Claims 1 to 7, wherein the analgesic is a therapeutic agent for pain accompanied by demyelinating disease.

12. The analgesic according to Claim 11, wherein the demyelinating disease is multiple sclerosis or Guillain-Bareé syndrome.

13. The analgesic according to any of Claims 1 to 12, wherein the analgesic is an injectable preparation.

14. The analgesic according to any of Claims 1 to 12, wherein the analgesic is an oral preparation.

15. The analgesic according to any of Claims 1 to 12, wherein the analgesic is a cyclodextrin inclusion complex.

16. The analgesic according to any of Claims 1 to 12, wherein the analgesic is an external preparation.

17. The analgesic according to Claim 16, wherein the external preparation is a patch preparation.

[Fig. 1]

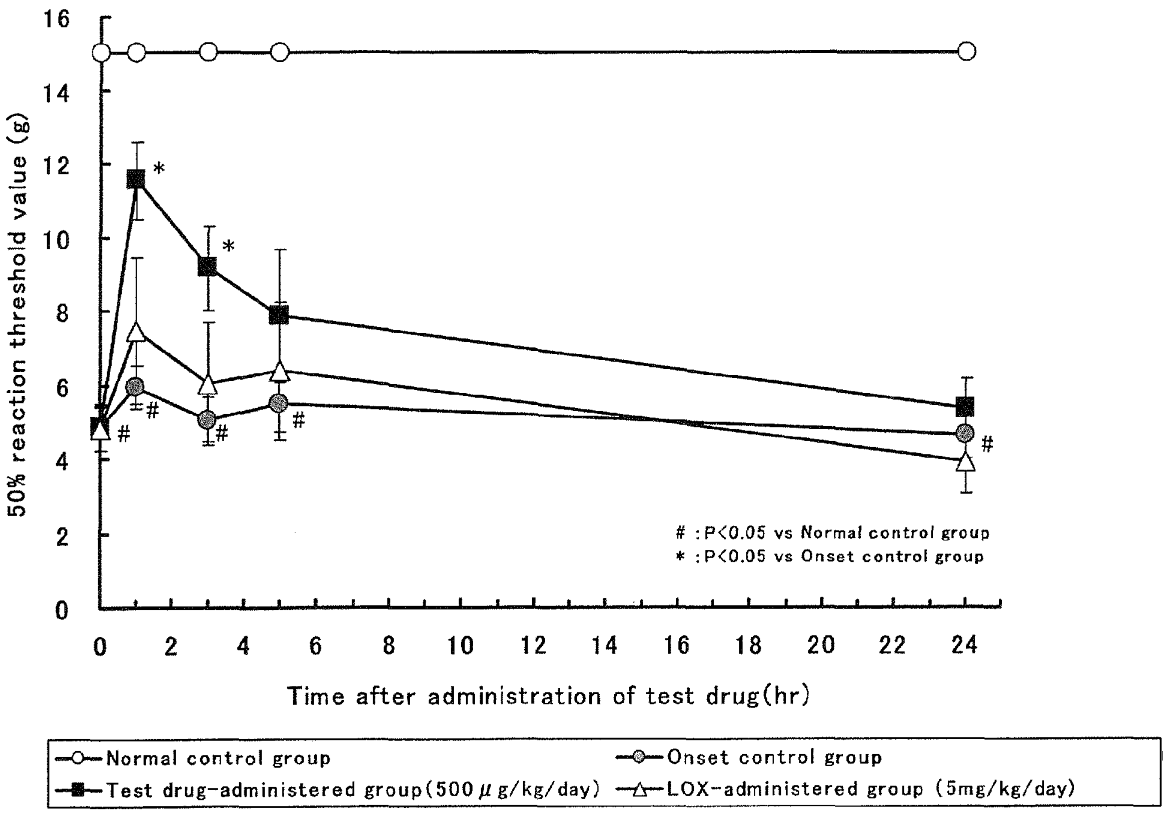

16
14
12
10
8
6
4
2
0
50% reaction threshold value (g)
0
2
4
6
8
10
12
14
16
18
20
22
24
Time after administration of test drug(hr)
# : P<0.05 vs Normal control group
* : P<0.05 vs Onset control group
Normal control group
Onset control group
Test drug-administered group(500 μg/kg/day)
LOX-administered group (5mg/kg/day)

[Fig. 2]

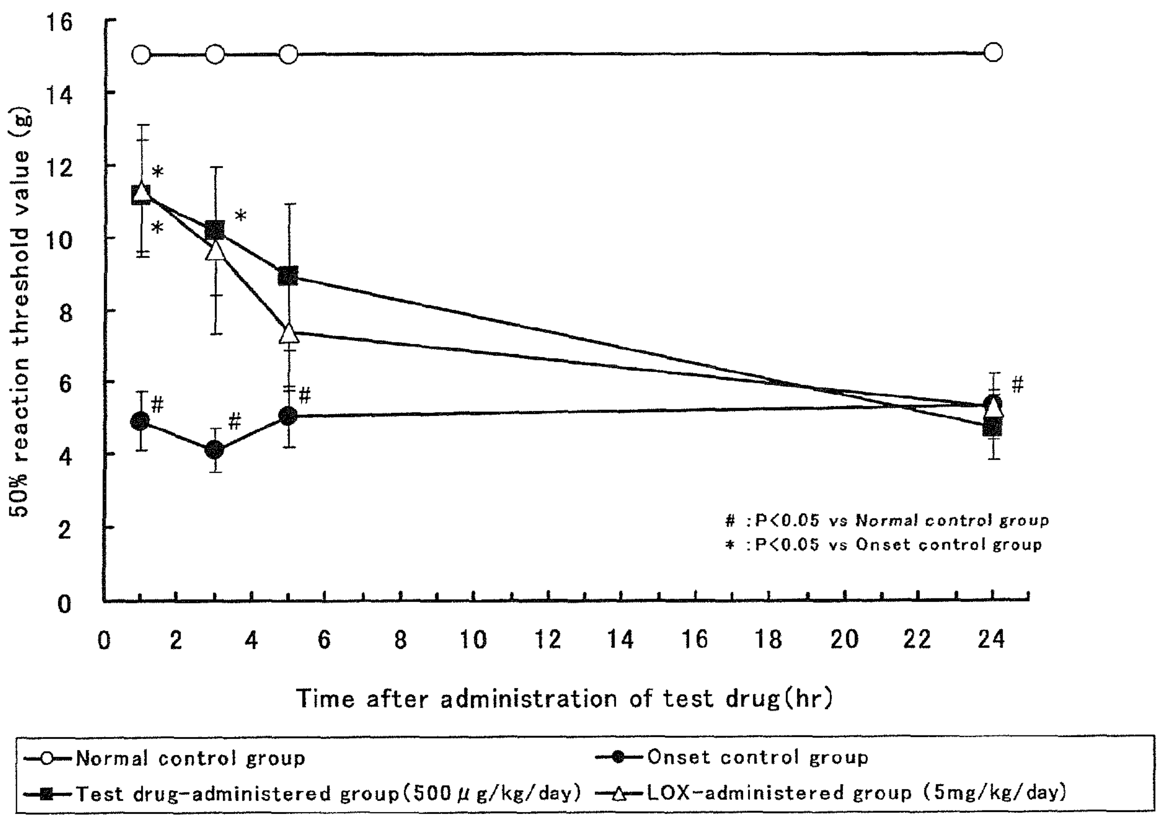
16
14
12
10
8
6
4
2
0
50% reaction threshold value (g)
0 2 4 6 8 10 12 14 16 18 20 22 24
Time after administration of test drug(hr)
# :P<0.05 vs Normal control group
* :P<0.05 vs Onset control group
Normal control group
Onset control group
Test drug-administered group(500 μg/kg/day)
LOX-administered group (5mg/kg/day)

[Fig. 3]

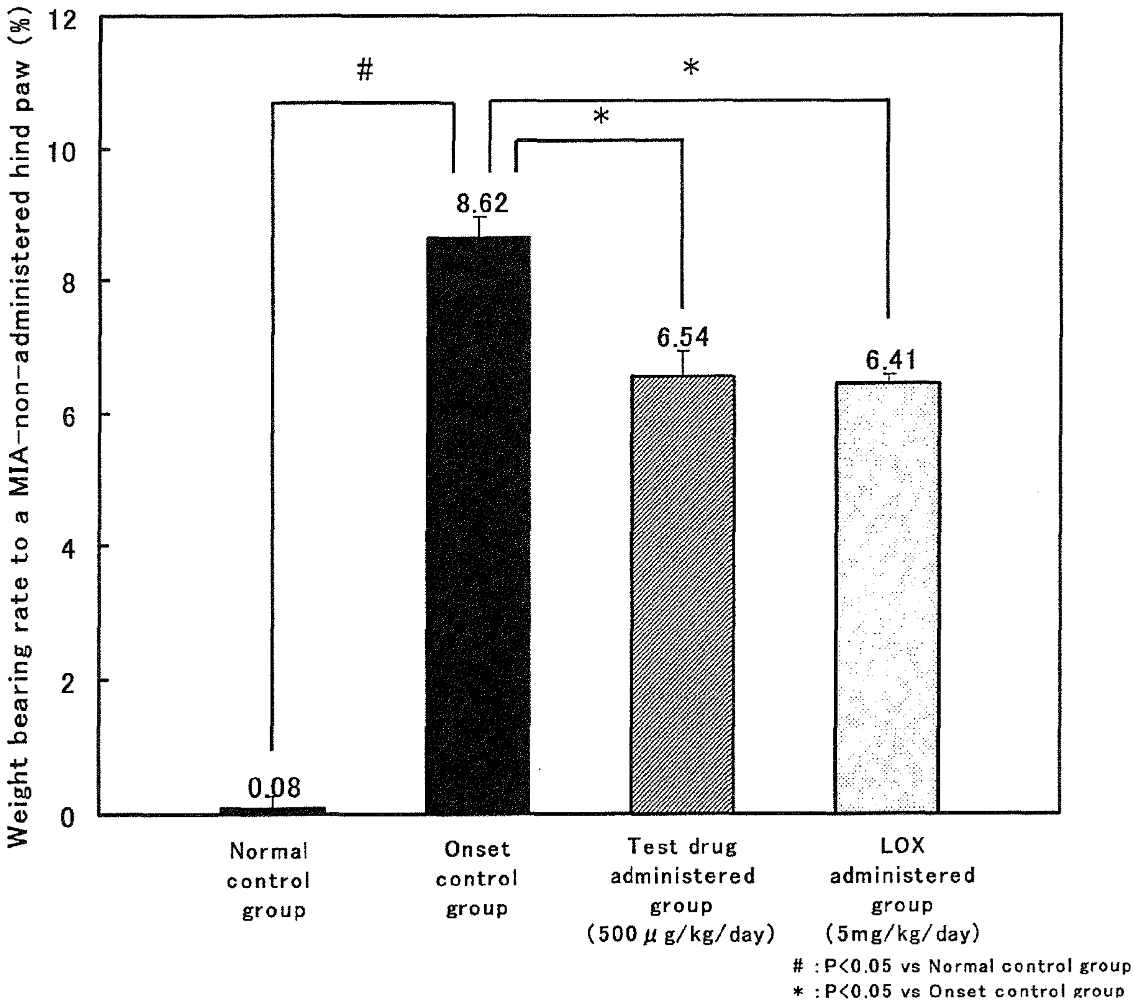
Weight bearing rate to a MIA-non-administered hind paw (%)
12
10
8
6
4
2
0
#
*
*
8.62
6.54
6.41
0.08
Normal control group
Onset control group
Test drug administered group (500 μg/kg/day)
LOX administered group (5mg/kg/day)
# : P<0.05 vs Normal control group
* : P<0.05 vs Onset control group

[Fig. 4]

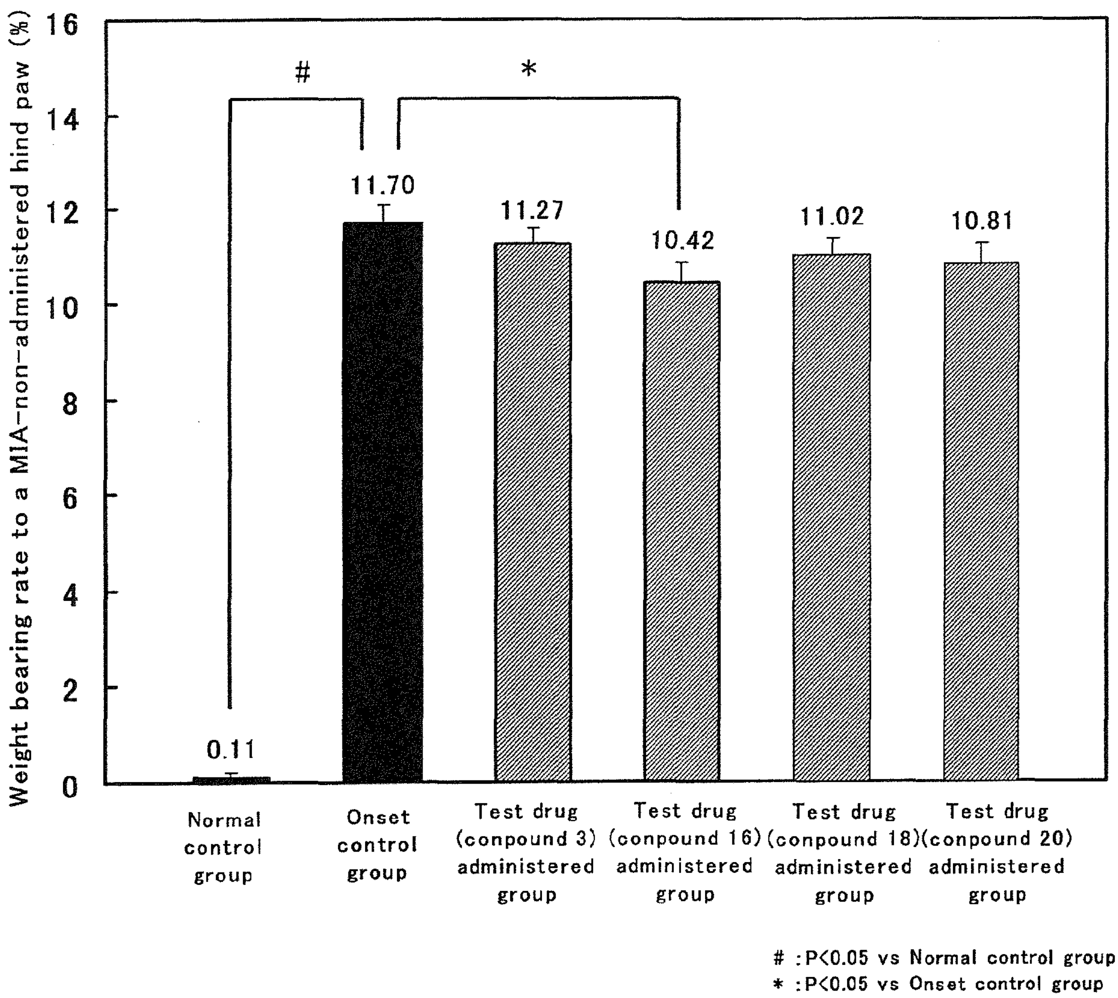
Weight bearing rate to a MIA-non-administered hind paw (%)
16
14
12
10
8
6
4
2
0
#
*
0.11
11.70
11.27
10.42
11.02
10.81
Normal control group
Onset control group
Test drug (conpound 3) administered group
Test drug (conpound 16) administered group
Test drug (conpound 18) administered group
Test drug (conpound 20) administered group
# :P<0.05 vs Normal control group
* :P<0.05 vs Onset control group

## INTERNATIONAL SEARCH REPORT

International application No.
PCT/JP2012/051939

A. CLASSIFICATION OF SUBJECT MATTER
*A61K31/201*(2006.01)i, *A61P29/00*(2006.01)i

According to International Patent Classification (IPC) or to both national classification and IPC

B. FIELDS SEARCHED

Minimum documentation searched (classification system followed by classification symbols)
A61K31/201, A61P29/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| A | WO 2009/038110 A1 (INST NAGOYA IND SCIENCE RES), 26 March 2009 (26.03.2009), entire text & EP 2204172 A1 & US 2010/0204498 A1 | 1-17 |
| A | WO 2003/000173 A2 (DPHARM LTD.), 03 January 2003 (03.01.2003), entire text & EP 1419129 A2 & JP 2005-500305 A | 1-17 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 February, 2012 (16.02.12) | 28 February, 2012 (28.02.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009038110 A


### Non-patent literature cited in the description


- **CHAPLAN et al.** *Journal of Neuroscience Methods,* 1994, vol. 53 (1), 55-63
- **DIXON et al.** *Annual Review of Pharmacology and Toxicology,* 1980, vol. 20, 441-462